(19) Europäisches Patentamt
European Patent Office
Office européen des brevets

(11) **EP 2 848 244 A1**

(12) **EUROPEAN PATENT APPLICATION**

(43) Date of publication:
**18.03.2015 Bulletin 2015/12**

(51) Int Cl.:
*A61K 9/20* (2006.01)    *A61K 31/554* (2006.01)

(21) Application number: **14183989.4**

(22) Date of filing: **08.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.09.2013 TR 201310894**

(71) Applicants:
- **Özsoy Erginer, Yildiz**
 **Istanbul (TR)**
- **Mesut, Burcu**
 **Istanbul (TR)**
- **Ozcelik, Esher**
 **Istanbul (TR)**
- **Aksu, Nese Buket**
 **Istanbul (TR)**
- **Araman, Ahmet**
 **Istanbul (TR)**

(72) Inventors:
- **Özsoy Erginer, Yildiz**
 **Istanbul (TR)**
- **Mesut, Burcu**
 **Istanbul (TR)**
- **Ozcelik, Esher**
 **Istanbul (TR)**
- **Aksu, Nese Buket**
 **Istanbul (TR)**
- **Araman, Ahmet**
 **Istanbul (TR)**

(74) Representative: **Mutlu, Aydin**
 **Invokat Intellectual Property Services**
 **Agaoglu My Office 212-D:241**
 **Basin Ekspres Yolu Tasocagi**
 **Cad. No: 3 Bagcilar**
 **34218 Istanbul (TR)**

(54) **Extended release tablet formulations of quetiapine**

(57)    The present invention relates to extended release compositions comprising quetiapine or pharmaceutically acceptable salts, solvates, polymorphs thereof. In the compositions presented within the scope of the invention, it is aimed to obtain an advantageous release profile by using xanthan gum polymer and to prepare the matrix structured tablets for this purposes which provide the desired release rate with the wet granulation method.

Fig. 1

EP 2 848 244 A1

**Description**

**THE FIELD OF THE INVENTION**

The present invention relates to an extended release formulation which comprising quetiapine or a pharmaceutically acceptable salt, solvate or polymorph thereof as well as a method characterized by using of xanthan gum polymer and preparing stable matrix structured tablets by wet granulation method. For this purpose, xanthan gum polymer was used as matrix agent to adjust the desired release rate.

**THE STATE OF THE ART**

Quetiapine fumarate has the chemical structure of 2-[2-(4-dibenzo [b,f][1,4] thiazepin-11-yl-1-piperazinyl) ethoxy] ethanol fumarate. Quetiapine Fumarate is an antipsychotic drug which is indicated in the treatment of schizophrenia, moderate to severe manic attacks in bipolar disorder, major depressive attacks in bipolar disorder, and prevention of recurrences in patients whose manic or depressive attacks in bipolar disorder were responded to quetiapine treatment. Although the posology of Quetiapine Fumarate changes according to diseases, it is recommended to be taken two times a day in the treatment of schizophrenia, major depressive disorder and in the treatment of moderate to severe manic attacks in bipolar disorder. In terms of the patient compliance, its extended release tablets are available in the market.

Quetiapine molecule is described in the patents EP 0240228 and US 4,879,288. As to the specific references related to the preparation and use of quetiapine fumarate, EP 240228, EP 282236, US 4,879,288 and WO 97/45124 can be exemplified. WO 2010/001413 discloses a formulation which consists of a non-gelling controlled release polymer and one or more excipients and it also includes a controlled release coating. In EP 1958617, a core comprising quetiapine and a binder and coated formulation was defined. In WO 97/45124, a formulation which consists of a gelling agent and one or more excipients is described.

In order to prepare the extended release tablets of quetiapine fumarate, it would be neccessary to select adjuvants and polimers which are stable and will provide the desired release rate. The selection of the polymer type and concentration is extremely important for providing the extended release tablets to give similar *in vitro* release profile with the reference product.

In the present invention, the extended release tablet formulations it was provided that quetiapine or a pharmaceutically acceptable salt thereof was released in 2 hours as maximum of 20-30%, and in 5 hours about 50%, and in 10 hours as a minimum 80% at pH 1.2.

**BRIEF DESCRIPTION OF THE FIGURES:**

Figure 1: The comparative *in vitro* release profiles of extended release film-coated tablet formulations (F1-F7) prepared by xanthan gum with the reference product (Serequel XR ® - AstraZeneca) are shown.

Figure 2: The comparative dissolution rate graphic of sample F7 according to the present invention at pH 1.2 is demonstrated.

Figure 3: The comparative dissolution rate graphic of sample F7 according to the present invention at pH 4.5 is demonstrated.

Figure 4: The comparative dissolution rate graphic of sample F7 of the invention at pH 6.8 is demonstrated.

**DETAILED DESCRIPTION OF THE INVENTION**

In this invention; it was aimed to provide a formulation which is developed comparatively with the reference drug by considering the different polymer types (Carbopol 974P and xanthan gum), polymer concentration, different granule sizes parameters of extended release formulations of quetiapine. For this reason, in order to achieve the desired release rate, formulations were prepared by use of different ratios of Carbopol 974P and xanthan gum polymer and the formulation which is equivalent to the commercially available reference drug based on *in vitro* release rate tests was determined according to International Commission of Harmonization (ICH) criteria.

It is easier to prepare the formulations of conventional dosage forms in the similar manner to the reference drug. But, providing extended release formulations of drugs is rather difficult. The reason is that there are many factors affecting the release rate of the drug from the formulation. In addition, in the drug release should be consistently ongoing during a predetermined time period. Among the advantages of extended release formulations; the following can be given i.e. the plasma level of the active substance remains constant in the desirable range in specified period, therapy can be achieved with the lower doses, accordingly the side and the toxic effects of active substance is becoming very low or totally eliminated, the fragmentation of active substances with short *in vivo* half life is prevented and their half lives are increased and the life quality of the patients are increased.

Extended release systems can be produced in many pharmaceutical forms. Some of these are oral systems (tablet, capsule, pellet, enteric coated tablet, film coated tablet etc.), mucoadhesive systems, ocular systems, nasal and pulmonary systems, transdermal systems, vaginal and rectal systems, and the systems carrying the drug to the colon. Commercially the first extended release pharmaceutical preparation in the world is the hard gelatin capsule including pellet which came onto the market in 1952 (Spansules, Smith Kline and French). Then, it was tried to develope the controlled drug release using different strategies and technologies.

The extended release oral delivery systems can be classified as matrix, membrane and osmotic pump controlled systems according to the preparation and release mechanisms. In matrix controlled systems, the active agent is homogeneously distributed in material(s) which control the release rate. It is usually prepared in the form of tablet, and in the preparation, the active substance is mixed with the insoluble powder polymers and it is tabletted directly or after granulation or after the preparation of solid dispersion with the polymer. In the granulation, known binding agents or the solvents in which the polymer is dissolved/swollen but the active ingredient is insoluble, can be used. Drug release from these systems occurs by the mechanism of drug diffusion and/or erosion. According to the structure of the polymer adjusting the release rate, these systems are divided into two as hydrophilic or hydrophobic matrix systems. The most commonly used hydrophilic matrix tablet is obtained by the basic tablet compression methods. The basic operations related to the preparation of matrices are not different from the methods used in the conventional tablet preparation. Mixing before granulation and coating the matrix tablets are complementary processes commonly used in the manufacture of matrix tablets. As active ingredient and release-limiting polymer, other excipients such as lubricants, diluents and adhesion inhibitors are also generally added to the formulation. Furthermore, these tablets are rendered more stable with a film layer which will totally eliminate the contact with the external environment.

It is also possible to obtain the extended release of the active ingredient without tabletting procedure, ie., granulating active ingredient with polymer and encapsulating it later or preparing pellets using a special machine (extruder, etc.).

Hydrophilic polymers used in the matrix systems are non-ionic soluble cellulose ethers (hydroxypropyl methylcellulose (HPMC), hydroxypropyl cellulose (HPC), hydroxyethyl cellulose (HEC)), non-ionic ethylene oxide homopolymers (polyethylene oxide (PEO) and its derivatives), water-soluble natural gums of polysaccharides (xanthan gum, sodium alginate, locust bean gum, etc.), crosslinked copolymers of acrylic acid with polyalkenyl alcohol and insoluble but swellable, in different particle sizes and in bond structures derivatives of high molecular weight homopolymers of its (Carbopol 974P, 934P, etc.), polyvinyl acetate and povidone mixture (Kollidon SR), Cross-linked high amylose starch, ionic methacrylate copolymers (Eudragit L30D etc.).

The inventors found that xanthan gum (CAS No: 11138-66-2) which is among the natural hydrophilic polymers used in matrix systems is more advantageous than Carbopol 974P in terms of giving results very close to the reference drug. The said gum is a high molecular weight polysaccharide which is formed by the pure culture fermentation of *Xanthomonas campestris* with the carbohydrate. Its molecular weight can be about $2 \times 10^6$ g mol$^{-1}$. In this context, the ratio of xanthan gum to the total formulation is in the range of 10-50% (w/w), more preferably 15-45% (w/w), most preferably 15-35% (w/w). The inventors determined a correlation between the amount and particle size of xanthan gum in terms of obtaining the desired release profile. In terms of Xanthan gum, it was observed that the ideal particle size distribution was obtained with the sieving of 0.5-2.0 mm, more preferably 0.5-1.5 mm in the range of 15-35% (w/w) in which very closest results to the reference drug are obtained. The best results were obtained by sieving in which its sieve opening 1 to 1.5 mm. With these modifications, the extended release formulations which most approximated to the reference drug were obtained.

In the pre-formulation experiments, when the active substance release rate profiles from the formulations which were prepared using Carbopol 974 polymer were compared with the release rate profiles of the reference drug, the former had lower f2-similarity factor (ICH) compared to the xanthan gum formulation (data not presented). Therefore, optimization studies were performed using formulations with xanthan gum.

Generic drugs in order to come into the market must show similar *in vitro* and *in vivo* performance to the reference drug. The success of the optimal formulation which will be determined by the *in vitro* performance tests to be done will be higher in *in vivo* bioequivalence study. Therefore, the *in vitro* performance of the formulation developed in R&D studies should show high similarity to the performance of the reference product.

The active substance used in the compositions of the present invention is preferably a pharmaceutically acceptable salts of quetiapine, more preferably quetiapine fumarate. Quetiapine fumarate is an antipsychotic drug which is indicated in the treatment of schizophrenia, moderate to severe manic attacks in bipolar disorder, major depressive attacks in bipolar disorder, prevention of recurrences in patients whose manic or depressive attacks in bipolar disorder were responded to quetiapine treatment. Although the posology of Quetiapine Fumarate changes according to diseases, it is recommended to be taken two times in a day in the treatment of schizophrenia, major depressive disorder and in the treatment of moderate to severe manic attacks in bipolar disorder. In order to minimize the fluctuations of the drug in plasma levels and to improve the patient compliance to the treatment, extended release preparations are preferred.

According to the invention, the presented extended release compositions consisting of Quetiapine and xanthan gum can be in any solid dosage form selected from tablet, film-coated tablet, capsule, pellet and granule. In the preferred embodiments of the invention, excipient(s) selected from at least one disintegrant, at least one binder, at least one filler and at least one lubricant may be included in the compositions of the invention.

According to a preferred embodiment of the invention, excipients apart from xanthan gum can be selected from MCC 101, lactose monohydrate, sodium citrate and magnesium stearate. The amount of magnesium stearate in the composition can be in a ratio of 0.5-1.5% (w/w) based on total formulation.

Again, according to another preferred embodiment, the tablet formulations is passed from a 1.0 mm or 1.4 mm sieve. In a more preferred embodiment, the tablet formulations of the invention are prepared by a process comprising the following steps:

a. homogeneously mixing of the active ingredient, the polymer and excipients,

b. performing wet granulation process,

c. passing the wet granules from the granulator,

d. drying the wet mass,

e. passing through the dried preparation (granules) from a 0.8 mm to 1.4 mm sieve,

f. adding magnesium stearate to the granule and mixing,

g. tablet compressing,

h. film coating of tablets.

Film-coating is a process of the protection of the tablet from the external environment and providing the ease of swallowing and it is a coating which does not affect the release.

Within the scope of the present invention, it is aimed to design a formulation of quetiapine, preferably of quetiapine fumarate with advantageous release profile. For this purpose, quetiapine tablets were prepared with different polymer concentrations and different granule particle sizes which formulations showed high similarity to the reference product (Serequel XR ® - AstraZeneca) in *in vitro* release rate tests.

**EXAMPLE: Preparation of Tablets**

**Preparation of Tablets Containing Quetiapine Fumarate**

In the preparation of the extended release tablets containing quetiapine fumarate, 3 different polymer ratios of xanthan gum polymers and two different granule particle size (preferably by passing through from a sieve of 1.0 and 1.4 mm) after wet granulation were used.

For the preparation of the extended release tablets containing quetiapine fumarate, primarily the polymer concentration ranges were determined. For this purpose, a concentration range of 15 to 45% (w/w) of xanthan gum was determined by evaluating in vitro release results.

The unit formulas belonging to the Quetiapine fumarate tablets prepared with xanthan gum were given in Table 1. The amount of quetiapine fumarate is 230 mg per tablet (equivalent to 200 mg quetiapine). In these tablets, preferably microcrystalline cellulose (MCC) 101 as disintegrant, pure water as binder (in a sufficient amount for granulation), lactose as the filler and sodium citrate as acid regulator, and magnesium stearate as lubricant were used.

**Table 1:** The contents of extended release tablet containing quetiapine fumarate

| CONTENT | F1* | F2** | F3* | F4** | F5* | F6** | F7* |
|---|---|---|---|---|---|---|---|
| **Quetiapine fumarate (mg)** (equivalent to 200.0 mg quetiapine) | 230.0 | 230.0 | 230.0 | 230.0 | 230.0 | 230.0 | 230.0 |

(continued)

| CONTENT | F1* | F2** | F3* | F4** | F5* | F6** | F7* |
|---|---|---|---|---|---|---|---|
| Xanthan gum (mg) | 93.0 | 93.0 | 155.0 | 155.0 | 217.0 | 217.0 | 119.37 |
| Microcrystalline cellulose (MCC) 101 (mg) | 100.0 | 100.0 | 100.0 | 100.0 | 100.0 | 100.0 | 100.0 |
| Lactose monohydrate (mg) | 52.63 | 52.63 | 52.63 | 52.63 | 52.63 | 52.63 | 52.63 |
| Sodium citrate (mg) | 88.86 | 88.86 | 88.86 | 88.86 | 88.86 | 88.86 | 88.86 |
| Magnesium stearate (mg) | 6.0 | 6.0 | 6.0 | 6.0 | 6.0 | 6.0 | 6.0 |
| Total tablet weight (mg) | 570.49 | 570.49 | 632.49 | 632.49 | 694.49 | 694.49 | 596.86 |
| *granule was sieved through a sieve of 1.0 mm. **granule was sieved through a sieve of 1.4 mm. | | | | | | | |

According to the preferred embodiments of the invention, the method for the preparation of the pharmaceutical formulation comprises without limitation the following steps;

i) weighing, sieving and homogeneously mixing of Quetiapine fumarate, MCC 101, lactose monohydrate, sodium citrate and xanthan gum,

j) performing the wet granulation process by using deionized water,

k) passing the wet mass through the granulator and drying the mass (at 45°C),

1) passing the dried mixture (granules) from preferably 1.0 mm or 1.4 mm sieve,

m) adding magnesium stearate to the granule and mixing them,

n) tablet compressing,

o) film coating of tablets (Film-coating is a process of the protection of the tablet from the external environment and providing the ease of swallowing and it is a coating which does not affect the release).

**The controls made on the tablets**

As a physical test; weight variation in tablets, thickness/height, % friability, hardness determination tests were performed. The RSD% value of the average weight variation of the tablets was found to be lower than 1.5%. It was determined that the average thickness values of the prepared tablets were between 5.36-6.01 mm and the hardness values of them were between 50N-150 N. The % friability test results of the tablets were found between 0.1-0.5% for all formulations and it complied with the limits given in the European Pharmacopoeia (1%).

As a chemical test; quantification of the active ingredients of the tablets and the dissolution rate tests were performed. In both tests, the validation studies belonging the analytical method used were done according to the criteria specified in the ICH Q2. HPLC device was used in the analytical method. The parameters of the analytical method used are given below.

Method: Reverse phase isocritic
Column: $C_{18}$ 250 x 4.6 mm, 5 $\mu$m
Mobile phase: Phosphate Buffer- acetonitrile (60:40 v/v) (pH 4.0)
Wavelength: 225 nm
Flow rate: 1.5 ml/min

The quantification results of the prepared extended release formulations (F1-F7) were found to be in the limits (95-105%).

In the scope of the present invention, USP-Paddle method, which is the standard method, was used in the *in vitro* dissolution rate studies of the extended release F1-F7 tablets with the reference product. As dissolution medium, solutions at three different pHs (1.2, 4.5 and 6.8) which simulates the gastrointestinal environment and recommended in ICH guidelines were used in an amount of 900 ml. In the dissolution rate test, the rotation speed of the paddle was adjusted to 50 cycles/min.

**Comparison of tablet formulations**

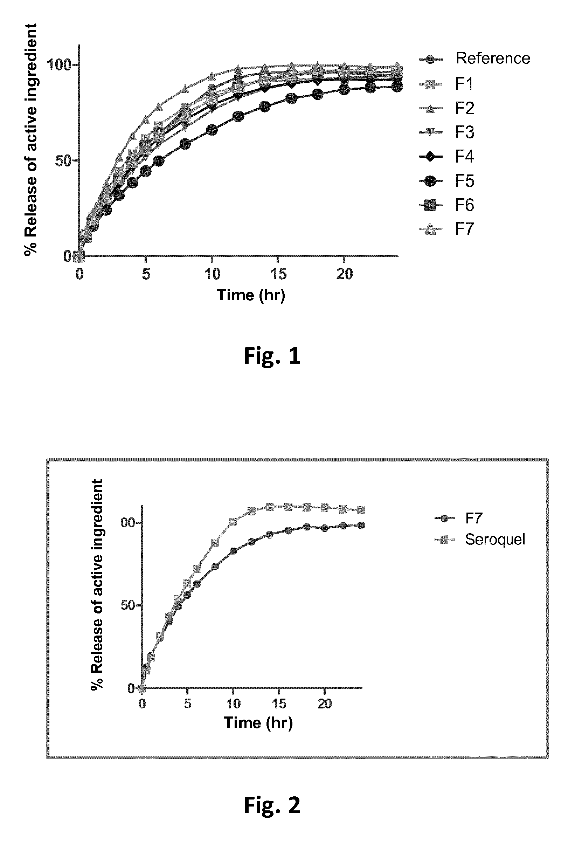

The release profiles obtained following the *in vitro* dissolution test result of extended release tablets prepared using xanthan gum polymer were given in comparison with the reference product (Serequel XR ® - AstraZeneca) in Figure 1.

The findings from the dissolution rate test belonging the formulations (F1-F7) were evaluated with the formula of "f2-similarity factor". The formula used in the calculation of the similarity factor (f2) is given below.

$$f_2 = 50 \times \log \left\{ \left[ \frac{1}{\sqrt{1 + \frac{1}{n} \sum_{j=1}^{n} (R_j - T_j)^2}} \right] \times 100 \right\}$$

If the result obtained from this formula is between 50-100, as this result approximates to the value of 100 in which two compared formulations are close to each other, it means that the degree of this proximity increases (***BCS Guidance FDA guidance***).

In vitro release of the formulations prepared for this determination were comparatively evaluated with the reference product using the USP-paddle method at three different pH (pH 1.2, pH 4.5 and pH 6.8) with 900 ml as prescribed by ICH guidelines and at 50 cycles/min.

According to the *in vitro* dissolution rate findings performed in the media of pH 1.2, pH 4.5 and pH 6.8, the formulation giving the the closest f2 value to the reference product was selected and the f2 values of the formulation calculated at these pH values were summarized in the following table.

**Table 2:** f2 values of F7 formulation

| pH | F2 Values |
|---|---|
| 1.2 | 76 |
| 4.5 | 50 |
| 6.8 | 62 |

Also the dissolution rate graphics obtained in each three medium belonging to this formulation were shown in Figures 2-4.

As a result of in vitro dissolution rate studies, the amount of active ingredient release from the formulation F7 at pH 1.2 is maximum of 20-30% at 2 hours, about 50% at 5 hours, and minimum of 80% at 10 hours. The comperative dissolution rate graphic with the reference product Seroquel® was given in Figure 2.

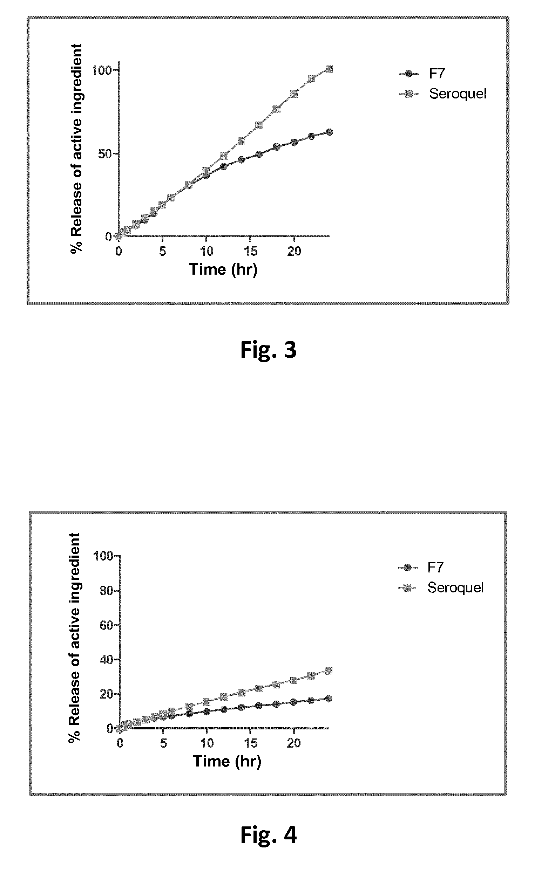

The amount of active ingredient released from the formulation F7 at pH 4.5 is minimum of 20% at 6 hours. Dissolution rate in comparison to the reference product Seroquel® chart is given in Figure 3.

The amount of active ingredient released from the formulation F7 at pH 6.8 is minimum of 5% at 6 hours. Dissolution rate in comparison to the reference product Seroquel® chart is given in Figure 4.

As a result of *in vitro* release studies, the F7 formulation which is a similar formulation to the reference product is a tablet of 596.89 g in weight and consisting of 230 mg quetiapine fumarate, 100 mg MCC 101, 52.63 mg lactose monohydrate, 88.86 mg sodium citrate and 119.37 mg Xanthan gum (see Table 1) . The granules of this tablet were sieved from a 1.0 mm sieve.

Consequently, it was shown that, with this invention, the extended release tablets of quetiapine fumarate can be prepared by the wet granulation method using xanthan gum which is a natural polymer. It was established in F1-F7 coded tablets that extended release can be obtained by the use of xanthan gum at a ratio of 15-45% (w/w), preferably 15-35% (w/w), more preferably 20% (w/w).

**Claims**

1. A Pharmaceutical composition in the extended release solid dosage form comprising quetiapine or pharmaceutically acceptable salt, solvate or polymorph thereof together with xanthan gum.

2. A composition according to claim 1 **characterized in that**; the pharmaceutical dosage form is selected from tablet, film-coated tablet, capsule, pellet and granule.

3. A composition according to claim 1 **characterized in that**; the said composition is in the extended release solid dosage form comprising of an excipient selected from at least one disintegrant, at least one binder, at least one filler, at least one lubricant.

4. A composition according to any of the preceding claims **characterized by** comprising xanthan gum in an amount of 10-50% (w/w) of the total weight.

5. A composition according to claim 4 **characterized in that**; the said composition comprises xanthan gum in a ration of 15-45% (w/w), preferably 15-35% (w/w), more preferably in a ratio of 20% (w/w), of the total weight.

6. A composition presented according to claim 3 **characterized in that**; it comprises magnesium stearate in a ratio of 0.5-1.5% (w/w) of the total weight of the said composition.

7. A composition according to claim 3 **characterized in that**; the said composition comprises lactose as a filler, microcrystalline cellulose as a disintegrant, sodium citrate as acid regulator and pure water as the binder.

8. A composition according to any of the preceding claims **characterized in that**; the *in vitro* active substance release of the composition is as follows when it is measured by using USP-paddle method in 0.1 N HCl, in 900 ml, at 50 cycle/min:

   After 2 hours, maximum 20-30% of quetiapine is released,
   After 5 hours, about 50% of quetiapine is released,
   After 10 hours, minimum 80% of quetiapine is released.

9. A composition according to any of the preceding claims **characterized in that**; quetiapine is in the form of fumarate salt.

10. A method for the preparation of a quetiapine pharmaceutical composition in extended release solid dosage form comprising subjecting quetiapine or its pharmaceutically acceptable a salt with xanthan gum and at least one excipient to wet granulation.

11. A method according to claim 10 **characterized in that**; quetiapine is in the form of fumarate salt.

12. A method according to claim 10 **characterized in that**; xanthan gum is added at a ratio of 15-45% (w/w), more preferably at a ratio of 15-35% (w/w) based on the total weight of the composition.

13. A method according to claim 10 **characterized in that**; the method further comprises the step of sieving the granule comprising quetiapine and excipients with a sieve of 0,8-1,6 mm

14. A method according to claim 13 **characterized in that**; the prepared dry granules are passed through a sieve of 1.0 mm.

15. A method according to claim 10 **characterized in that**; the said method further comprises the step of tablet compressing.

**Fig. 1**

**Fig. 2**

**Fig. 3**

**Fig. 4**

Europäisches
Patentamt

European
Patent Office

Office européen
des brevets

# EUROPEAN SEARCH REPORT

Application Number

EP 14 18 3989

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DEEPTHI B; MANIKIRAN S S; LAKSHMI PRASANNA L; RAMA RAO N: "AN INVESTIGATION OF HYDROPHILIC NATURAL GUMS IN THE FORMULATION OF QUETIAPINE FUMARATE MATRIX TABLETS", INTERNATIONAL JOURNAL OF PHARMACY AND PHARMACEUTICAL SCIENCES : IJPPS, vol. 4, no. Suppl. 4, 1 September 2012 (2012-09-01), pages 569-574, XP55165647, IN ISSN: 0975-1491 * abstract * * page 569, right-hand column, paragraphs 2,3; figures 2b,2d; tables 1,2,4,5 * * page 573, right-hand column, last paragraph - page 574, left-hand column, paragraph 2 * | 1-15 | INV. A61K9/20 A61K31/554 |
| X | US 2009/264408 A1 (GULATI INDER [IN] ET AL) 22 October 2009 (2009-10-22) * paragraphs [0044] - [0049], [0072] - [0079]; examples 2,7 * | 1-15 | |
| A | WO 2010/082220 A2 (TORRENT PHARMACEUTICALS LTD [IN]; VAYA NAVIN ISHWARLAL [IN]; SONI NARE) 22 July 2010 (2010-07-22) * the whole document * | 1-15 | TECHNICAL FIELDS SEARCHED (IPC) A61K |
| A | EP 2 153 834 A2 (FARMAPROJECTS S A [ES]) 17 February 2010 (2010-02-17) * the whole document * | 1-15 | |

The present search report has been drawn up for all claims

| Place of search | Date of completion of the search | Examiner |
|---|---|---|
| Munich | 29 January 2015 | Toulacis, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 14 18 3989

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2015

| Patent document cited in search report | | Publication date | Patent family member(s) | | Publication date |
|---|---|---|---|---|---|
| US 2009264408 | A1 | 22-10-2009 | NONE | | |
| WO 2010082220 | A2 | 22-07-2010 | EP | 2373319 A2 | 12-10-2011 |
| | | | US | 2011280938 A1 | 17-11-2011 |
| | | | WO | 2010082220 A2 | 22-07-2010 |
| EP 2153834 | A2 | 17-02-2010 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0240228 A
- US 4879288 A
- EP 240228 A
- EP 282236 A
- WO 9745124 A
- WO 2010001413 A
- EP 1958617 A